# EUROPEAN PATENT APPLICATION

(11) **EP 3 511 347 A1**
(43) Date of publication of application: **17.07.2019**
(21) Application number: 17915822.5
(22) Date of filing: 28.08.2017
(51) Int. Cl.: C07K 19/00, C07K 16/10, C12N 15/13, C12N 15/62, C12N 15/867, C12N 5/10, A61K 35/17, A61P 31/18

(54) **CONSTRUCTION OF RECOMBINED GENE OF CHIMERIC ANTIGEN RECEPTOR FOR TREATMENT OF HIV INFECTION, AND APPLICATION THEREOF**

(30) Priority: 28.06.2017 CN 201710507860
(71) Applicant: Wuhan Bio-Raid Biotechnology Co., Ltd, Wuhan, Hubei 430075 (CN)
(72) Inventor: ZHANG, Tongcun, Wuhan Hubei 430075 (CN); GU, Chaojiang, Wuhan Hubei 430075 (CN); LIAO, Xinghua, Wuhan Hubei 430075 (CN)
(74) Representative: Hanna Moore + Curley
(86) International application number: PCT/CN2017/099261
(87) International publication number: WO 2019/000620

(57) **Abstract**

The present invention relates to the technical field of immunotherapy of infectious diseases, in particular to construction and application of a recombinant gene for chimeric antigen receptor (CAR) for treating HIV infection. The present invention provides a single-chain antibody ScFv capable of recognizing gp120 on the surface of an HIV virus-infected cell, which is obtained by tandemly ligating light chain and heavy chain variable regions of an antibody against gp120 on the surface of the HIV virus-infected cell; the single-chain antibody is made into a chimeric antigen receptor (CAR), the CAR encoding gene is transferred into a plasmid vector, and the lentiviral vector into which the CAR encoding gene is transduced is transduced into CD8⁺ T lymphocytes. The obtained CAR-T lymphocytes have been found to have significant activity in inhibiting and killing HIV-infected cells in both in vitro and in vivo experiments, which can be used as an active ingredient for preparing a drug against HIV infection, thereby having a good application prospect.

## Description

### Technical Field

The invention relates to the technical field of immunotherapy of infectious diseases, in particular to construction and application of a recombinant gene for a chimeric antigen receptor for treating HIV infection.

### Background Art

AIDS is a major infectious disease that threatens the safety of human life caused by human immunodeficiency virus type 1 (HIV-1) infection. According to the latest statistics of the World Health Organization, more than 39 million people have died of AIDS since it was discovered by the end of 2014, and 37 million people are still living with HIV worldwide. The number of people living with HIV in China has increased year by year, and the total number of patients has exceeded one million. There is currently no effective vaccine, and existing drugs cannot completely cure AIDS.

Highly active antiretroviral therapy (HAART) is the first revolution in the history of HIV/AIDS treatment, which greatly reduces the incidence and mortality of HIV/AIDS, significantly prolongs patient life and even reduces HIV transmission. However, there are also many challenges: 1) patients must take their medicines for life, which requires expensive economic costs; 2) serious toxic and side effects; 3) emergence of drug-resistant strains of HIV; and 4) more importantly, cART cannot completely clear the virus, mainly because the drug is only effective against the virus in the replication, and is ineffective for the latent virus "reservoir" established by HIV in the early stages of infection. Once the antiretroviral therapy is interrupted, the integrated provirus in the virus reservoir is reactivated and viremia will rebound rapidly in almost all patients.

The high variability of HIV virus in its transmission and reproduction has made the previous effective AIDS drugs ineffective, therefore,"cocktail therapy"came later, which is to combine multiple antiviral drugs for HAART. However, cocktail therapy also has its limitations. In light of the current frontier and hotspot in the international research on AIDS, researchers generally believe that the main reason why HIV cannot be cured is that HIV establishes a hidden virus "reservoir" in the body at the very early stage of infection, that is, Resting memory CD4+ T lymphocyte.

One of the major challenges in finding a cure for AIDS is how to reactivate the latent HIV to be recognized and shuffled by the body's immune system (shock and kill). At the same time, the study found that even in the body of infected patients receiving cART therapy, there is also irreversible immune damage, especially the reduction of the number of cytotoxic T lymphocyte (CTL) and functional defects, indicating that the immune system reconstructed by antiretroviral therapy will not effectively clear those activated cells, and it is necessary to enhance the clearance of HIV reservoirs by a method of jointly enhancing the body's HIV-specific immune response. Therefore, the exploration of the formation and activation strategies of the HIV latent virus "reservoir" and the reconstruction of the body's immune function are all extremely valuable research directions in the era of curing the HIV.

In recent years, chimeric antigen receptor (CAR)-based tumor immunotherapy technology has created a new way to kill tumor cells. Due to its high affinity and MHC (Major histocompatibility complex) independence and other advantages, especially the third-generation CAR formed by coupling two CD28 and 4-1BB, and CD3ζ can enhance the tumoricidal ability of T cells and prolong their survival time in vivo, thus achieving encouraging results in tumor immunotherapy such as leukemia and lymphoma, etc. In fact, as early as 1994, Roberts et al. tried to treat HIV infection with CAR-T cells. They selected a CD4 sequence as a single-chain antibody for binding to gp120 on the surface of the infected cells. Although it had the function of partially killing infected cells and after years of efforts, it ended in failure. The main reasons are as follows: 1. the transduction efficiency using a retroviral vector is low, in order to obtain sufficient CAR-T cells for reinfusion, excessive expansion in vitro results in cell death and loss of CAR molecules after reinfusion; and 2. the CAR molecular design itself has defects, in which the CD4 domain may cause transduced CTLs to be infected by HIV, or the virus-infected cells may escape the killing of CAR-T cells by down-regulating the expression of CD4 molecules.

### Technical Problem

In order to overcome the above-mentioned deficiencies of the prior art, the present invention provides a novel single-chain antibody ScFv capable of recognizing gp120 on the surface of an HIV virus-infected cell and a chimeric antigen receptor (CAR) made of the single-chain antibody, which is called an N6-CAR molecule.

Another object of the present invention is to provide an expression vector capable of expressing the above N6-CAR and an N6-CAR vector gene-modified CD8⁺ T lymphocyte.

Still another object of the present invention is to provide a use of the N6-CAR molecule-modified CD8⁺ T lymphocyte for the preparation of a medicament against HIV infection.

### Technical Solution

In order to achieve the above objects, the present invention is achieved by the following technical solutions:
providing a single-chain antibody ScFv, which is capable of recognizing gp120 on the surface of an HIV virus-infected cell, is obtained by tandemly ligating light chain and heavy chain variable regions of an antibody against gp120 on the surface of the HIV virus-infected cell, and serves as an extracellular binding domain of the entire CAR molecule, and its amino acid sequence is shown in SEQ ID NO. 2.

The present invention also provides an encoding gene encoding the above single-chain antibody ScFv, the nucleotide sequence of which is shown in SEQ ID NO. 1.

The present invention also provides a chimeric antigen receptor for treating HIV infection, which is obtained by sequentially splicing a signal peptide, the single-chain antibody ScFv provided by the present invention, CD8 hinge, transmembrane domain of cluster of differentiation CD28-TM and intracellular domain ICD, 4-1BB and ζ chain of cluster of differentiation 3 CD3 from N-terminal to C-terminal, and the amino acid sequence of the obtained chimeric antigen receptor is as shown in SEQ ID NO. 4.

The present invention also provides an encoding gene encoding the above chimeric antigen receptor N6-CAR, the nucleotide sequence of which is shown in SEQ ID NO. 3.

An expression vector containing and capable of expressing an encoding gene of the chimeric antigen receptor N6-CAR having the amino acid sequence of SEQ ID NO. 4, the vector is a PTK-EF1α-N6 vector obtained by transformation by using a PTK881 vector as a backbone and replacing a CMV promoter with an EF1α promoter, the nucleotide sequence of which is shown in SEQ ID NO. 5.

The present invention provides a genetically modified CD8⁺ T lymphocyte, which is a genetically engineered T-lymphocyte capable of expressing a chimeric antigen receptor obtained by transducing a lentiviral vector of a 293T cell transfected with the PTK-EF1α-N6 expression vector into a CD8⁺ T lymphocyte. The N6-CAR-modified CD8⁺ cells have a more significant killing effect on cell lines expressing gp120.

Further, the genetically modified CD8⁺ T lymphocyte is prepared by the following methods: (1) isolating PBMC from peripheral blood and then using magnetic beads for positive screen to obtain CD8⁺ T cells, stimulating with anti-CD3/28 magnetic beads (ratio of cell to magnetic bead: 1:3) for 12 hours, adding a lentivirus recombinant with a N6-CAR molecule for infection for 4 hours, and then supplementing fluid (MOI = 5); starting from the third day after virus infection, counting cells and supplementing the culture medium according to cell state and proliferation, adjusting the cell concentration to 0.6 x 10⁶/ml, and supplementing 100 U/mL IL-2; and further expanding the cells until the number of cells for reinfusion is met.

The present invention also provides a use of the above N6-CAR gene-modified CD8⁺ T lymphocyte, and the N6-CAR gene-modified CD8⁺ T lymphocyte is used to prepare a live cell drug against HIV infection.

### Summary of the Invention

Beneficial effect of the present invention: (1) the present invention overcomes the shortcomings of the early design, utilizes a broad-spectrum neutralizing antibody capable of highly specifically binding to the viral protein Gp120 as a ScFv, and can bind to 98% of HIV-1 virus strains, thereby increasing the broad spectrum of the CAR-T cells; (2) the present invention uses a PTK plasmid containing a SIN (self-inactivating) structure to produce a lentiviral vector with increased safety, while modifying a CAR molecule into a bi-stimulatory molecule to increase the expansion and survival characteristics of N6-CAR-T cells, thereby increasing clinical efficacy and safety; and (3) the genetically modified CD8⁺ T lymphocyte capable of expressing a chimeric antigen receptor in the present invention has been found to have significant activity for inhibiting and killing HIV virus in both in vitro and in vivo experiments, and is capable of producing an anti-HIV infection drug as an active ingredient.

### Brief Description of the Drawings

FIG. 1 is a schematic diagram showing the structure of the chimeric antigen receptor against HIV infection constructed by the present invention;
FIG. 2 is a schematic diagram showing the structure of the PTK-EF1α-N6 lentiviral vector constructed by the present invention;
FIG. 3 shows the detection of the expression level (A) of N6-CAR in the transduced CD8⁺ T lymphocytes and its functional proliferative potential (B) after stimulation in the present invention;
FIG. 4 shows the detection of the activity of the N6-CAR transduced CD8⁺ T lymphocytes of the present invention in killing HIV-infected cells in vitro;
FIG. 5 shows the detection of the activity of the N6-CAR transduced CD8⁺ T lymphocytes of the present invention in inhibiting virus under co-culture conditions; and
FIG. 6 shows the detection of the activity of the N6-CAR transduced CD8⁺ T lymphocytes of the present invention in killing HIV-infected cells in humanized mice.

### Detailed Description of the Invention

The examples are shown to illustrate certain embodiments of the present invention and are not to be construed as limiting the scope of the present invention. The disclosure of the present invention can be modified from the materials, methods, and reaction conditions, all of which are within the spirit and scope of the present invention.

### Example 1:

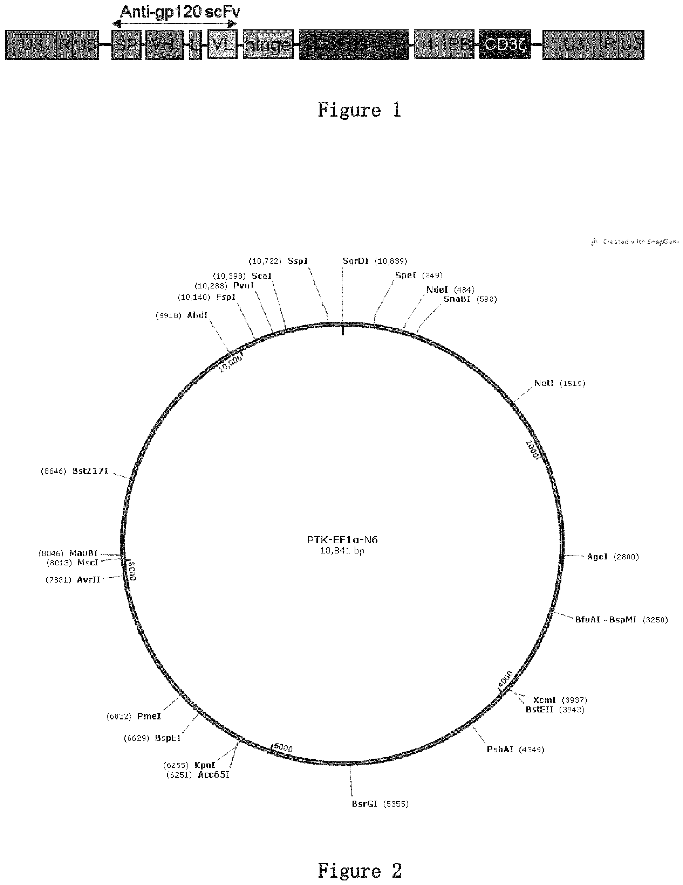

The present invention provides a chimeric antigen receptor (CAR) recombinant gene for treating HIV infection and a construction method thereof, and the specific splicing method is: sequentially splicing a signal peptide, a single-chain antibody ScFv capable of recognizing gp120 on the surface of an HIV virus-infected cell, CD8 hinge, transmembrane domain of cluster of differentiation CD28-TM+ICD, 4-1BB and ζ chain of CD3 (cluster of differentiation 3), and finally obtaining a complete chimeric antigen receptor (CAR) molecule capable of treating HIV, the amino acid sequence of which is SEQ ID NO. 4, and the structure of which is shown in FIG. 1; and the nucleotide sequence of a gene encoding the chimeric antigen receptor (CAR) is shown in SEQ ID NO. 3.

The amino acid sequence of the chimeric antigen receptor-derived single-chain antibody ScFv for treating HIV infection is shown in SEQ ID NO. 2, and the single-chain antibody is obtained by tandemly ligating light chain and heavy chain variable regions of an antibody against gp120 on the surface of the HIV virus-infected cells, and the nucleotide sequence of a gene encoding the same is shown in SEQ ID NO. 1.

The structural design of the CAR molecule of the present invention is described in detail by the chimeric antigen receptor (CAR) molecule shown in SEQ ID NO. 4. The N-terminal of the sequence of the CAR molecule is a CAR-derived ScFv sequence, which can specifically recognize gp120 on the surface of the HIV virus-infected cells; and the C-terminal of the sequence of the CAR molecule is based on a third-generation CAR structure, comprising CD8 hinge, CD28TM+ICD, 4-1BB and CD3ζ intracellular domain which are tandemly ligated. The ScFv and intracellular signal molecule are linked by the transmembrane domain of the CD28 molecule. In the above structure, each fragment can function as follows: the signal peptide can secrete CAR into the extracellular, the CD28TM+ICD anchors the CAR of the present invention to the cell membrane; the ScFv specifically recognizes gp120 on the surface of the HIV virus-infected cells; and CD3ζ is an intracellular signal activating sequence, which activates a signal after the ScFv binds to the antigen, and initiates killing activity of lymphocyte.

### Example 2: Recombinant construction of PTK-EF-1α-N6 plasmid expression vector with CAR molecule

The CAR was synthesized according to the sequence shown in SEQ ID NO. 4, and the full-length CAR encoding gene was inserted into the target expression vector by a seamless recombinant cloning technique (see FIG. 2). After several experiments, the preferred plasmid vector was a PTK-EF1α-N6 vector (the nucleotide sequence of which is shown in SEQ ID NO. 5) obtained by transformation by by using a PTK881 vector as a backbone and replacing the CMV promoter with the EF1α promoter. Finally a recombinant plasmid PTK-EF1α-N6 vector into which the CAR gene was inserted and was capable of expressing CAR was obtained. Its nucleotide sequence is shown in SEQ ID NO. 6.

The virus packaging steps are as follows:
1) Two centrifuge tubes each containing 16 ml of DMEM culture fluid were taken, one of which was added with 960 µg of PEI, and the other tube was added with 320 µg of premixed PTK881 vector plasmid, shaken with vortex, and equilibrated at room temperature for 10 minutes.
2) A 10 ml pipette was used to blow up the culture medium mixed with PEI, and the culture medium mixed with the plasmid was added to PEI drop by drop, and the mixture was incubated at room temperature for 30 minutes.
3) To a T175 bottle, 3 ml of fetal calf serum was added, and the PTK-EF1α-N6 vector plasmid mixed with PEI was added thereto, and then the culture medium in the multi-layer cell culture flask was poured into the T175 bottle, which was shaken up and down and left and right to make the culture medium mixed with the plasmid, and finally the culture medium in the T175 bottle was poured back into the multi-layer cell culture flask. The supernatant was harvested after incubation for 3 days in an at incubator at 37°C and 5% CO₂. The collected supernatant was centrifuged at 4000 rpm (3000 g) for 30 min to remove 293T cell debris.
4) The lentiviral liquid supernatant was filtered through a 0.22 µm filter, dispensed into 250 ml centrifuge bottles, and centrifuged at 30,000 g for 2.5 hours at 4°C. After centrifugation, the centrifuge bottle was carefully transferred to a biosafety cabinet, and the supernatant was removed by a vacuum pump to leave the precipitate. The T cell culture medium was added with 500 µl/centrifuge bottle. The precipitate was dispersed evenly mixed by blowing with a gun to obtain a lentiviral vector containing the N6-CAR molecule, which was immediately used or dispensed and stored at -80°C.

### Example 3: Preparation of CD8⁺ T cells (CAR-T cells) capable of expressing a chimeric antigen receptor

### Step 1: Isolation of patient PBMC cells

60-80 ml of human peripheral blood samples were collected and shaken while collecting to make the peripheral blood and anticoagulant fully mixed;
the peripheral blood was transferred to a 50 ml centrifuge tube, and diluted with DPBS buffer in a ratio of 1:1 and evenly mixed. The diluted blood sample was slowly added to a centrifuge tube of 15 ml of human lymphocyte separation liquid at room temperature. The method is as follows: the blood sample was pipetted with a 10 ml pipette, the pipette was extended to 0.5 cm above the liquid level of the separation liquid, and the blood sample was naturally slipped onto the separation liquid surface, and then the blood sample was gently added, and the liquid surface was taken carelessly not to be broken.

The mixture was centrifuged for 30 minutes with slow raising speed and slow reducing speed.

After centrifugation is completed, the centrifuge tube was clearly layered from bottom to top: red blood cell layer, granulocyte layer, Ficoll layer, mononuclear cell layer and plasma layer. The plasma layer was pipetted to about 5 mm from the white film layer and discarded. All the liquid above the red blood cell layer was carefully pipetted into a centrifuge tube, diluted with PBS, and evenly mixed with the cell suspension in a volume ratio greater than 1:3.

After centrifugation (1600 r/min) for 5 min, the cells were resuspended in PBS and evenly mixed, and a small number of cells were taken for counting.

After centrifugation at 300 g (1200 r/min) for 5 min, the supernatant was sent for sterility testing.

### Step 2: CD8⁺ T cell sorting

(1) PBMC cells in step 1 were resuspended in 30 ml of normal saline and sampled and counted (after sampling, normal saline was supplemented to 50 ml, the mixture was evenly mixed and centrifuged at 500 g for 10 min at 18°C with fast raising speed and fast reducing speed, the supernatant was removed). After counting, the cells and buffer were mixed according to 10⁷ cells / 80 µL buffer (if the supernatant was not completely removed, it was recommended not to add buffer). The CD8 Microbeads were added according to 10⁷ cells / 20 µL of CD8 Microbeads, and the mixture was incubated for 15 min at 4-8 °C.
(2) After the incubation is completed, the cells were washed with 1 to 2 ml of buffer per 10⁷ cells, and the mixture was centrifuged at 500 g for 10 min.
(3) Up to 10⁸ cells were resuspended in 500 µL of buffer (if the number of cells was large, more buffer may be used).
(4) A Miltenyi special LS column was placed on a magnetic stand. After washing the LS column with 3 ml of buffer, the cell suspension was added to the LS column and drained. The LS column was washed three times with 3 ml of buffer and each time it was drained. The LS column left the magnetic stand. 5 ml of buffer was added to the LS column and the labeled cells were flushed out with a piston (flushing could be done twice to ensure that the labeled cells can be flushed out).
(5) After the CD8⁺ T cells were flushed out, they were resuspended to 30 ml with normal saline, sampled and counted, and centrifuged at 500 g for 10 min at 18°C to obtain a cell pellet, which could be used for culture.

### Step 3: Activation of CD8⁺ T cells

(1) The CD8⁺ T cells in step 2 were counted and added to a culture flask at a density of 2×10⁶/ml, and evenly mixed and placed in a CO₂ incubator and incubated for 2 hours.
(2) The culture flask was taken out and gently shaken to float the suspended cells deposited at the bottom. The culture medium was pipetted into a centrifuge tube. The culture flask was washed with a small amount of culture medium to collect all the suspended cells, which were evenly mixed and counted.
(3) The cell concentration was adjusted according to the cell count, and the cells were inoculated into a culture flask at a concentration of 1.2×10⁶/ml (100 to 120 ml in a T150, 50 to 60 ml in a T75, and 15 to 29 ml in a T25). CD3/CD28 magnetic beads were added thereto at a ratio of cell to magnetic bead 1:3 (before adding, the magnetic beads were washed three times with the culture medium to remove the preservation solution). 100 U/mL IL-2 was added thereto. After evenly mixing, the culture flask was placed in a CO₂ incubator for cultivation, and the cells were collected.

### Step 4: CD8⁺ T cells were transduced with N6-CAR molecule to prepare CAR-T cells

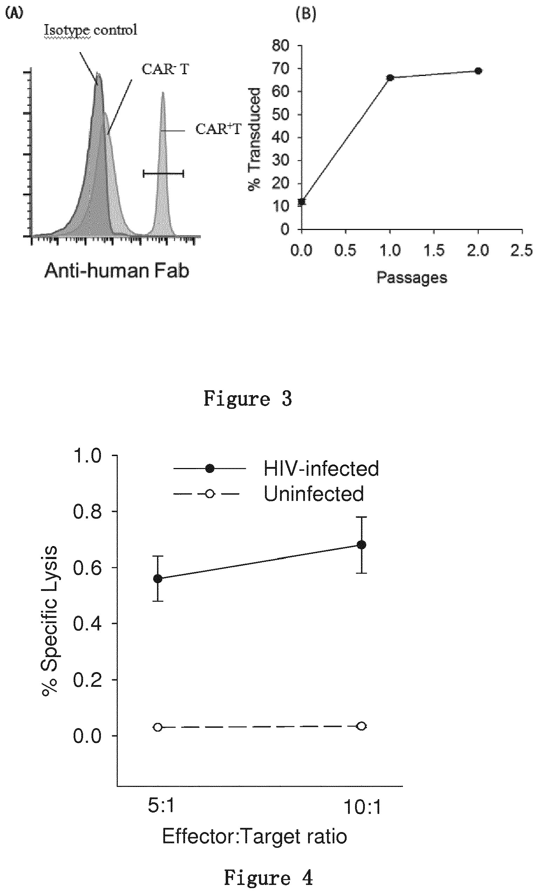

After adding the magnetic beads for 12 hours, an appropriate amount of the CD8⁺ T cell suspension in a good condition in the step 3 was placed in a centrifuge tube and centrifuged at 300 g for 5 minutes, and the supernatant was discarded. The chimeric antigen receptor (CAR) lentiviral vector was added at a ratio of 1 x 10⁶ / ml of cells, while adding Polybrene at a final concentration of 4 µg/ml, and evenly mixed. The cell suspension was incubated in a small volume at 37°C. After incubating for 4 hours, an appropriate amount of T cell complete medium was supplemented for culture. On the third day of cell culture, the cells were counted, and the culture medium was supplemented according to the state and proliferationof the cells. The cell concentration was adjusted to 0.6 x 10⁶/ml, and 100 U/mL IL-2 was supplemented. On the 5th day of cell culture, the cells were evenly mixed and transferred into a centrifuge tube. The magnetic beads were removed on a magnetic stand. The cells were counted, and supplemented with culture medium and 100 U/mL IL-2. The cell density was adjusted to 0.6 x 10⁶/ml and the culture was continued. The expression of SCFV was detected by flow cytometry. At the same time, some CD8⁺ T lymphocytes were stimulated by goat anti-human Fab antibody and serially passaged to determine the self-amplification ability of anti-gp120 CAR-transduced CD8⁺ T lymphocytes. The results are shown in FIG. 3.

The results showed that 40% of CD8⁺ T cells expressed CAR molecules after five days of culture of Anti-gp120 CAR virus transduced cells. When the anti-human Fab antibody specifically bound to the CD8⁺ T cell-expressed CAR molecule, it could effectively and dose-dependently activate cell proliferation. As the number of passages increased, the proportion of CAR molecule positive cells population gradually increased.

### Example 4: Detection of activity of CAR-T cells in killing HIV-infected cells in vitro

In order to further detect the function of N6-CAR, N6-CAR-T cells were mixed and cultured with two HIV-1 infected cell lines, H9-NL4-3 and H9-NDK, respectively. The cell killing experiments were performed in 96-well plates in U-bottom. First, the HIV-infected cell line H9 and the negative control cells were labeled with Calcein-AM. 100 µl (contained target cell number: 10⁴) was placed in a 96-well plate, and 100 µl of gradient-diluted CAR-T cells were added to the corresponding 96-well plates to ensure a range of the ratio of effective cells to target cells of 5:1 to 10:1 with a final volume of 200 µl per well. The mixture was centrifuged at 200 g for 30 minutes at room temperature and incubated at 37°C for 2-3 hours. The supernatant was obtained by centrifugation to measure fluorescence and calculate the percentage of lysis, which was used to determine the cytotoxicity of N6-CAR-T cells against HIV-infected cells. The experimental results are shown in FIG. 4.

The results showed that the N6-CAR-T cells significantly killed the target cell lines infected with two HIV strains in a dose-dependent manner in a range of the ratio of effective cells to target cells of 5:1 to 10:1, but had no obvious killing effect on the control target cells, indicating that the effect of N6-CAR-T cells in killing target cells is specific for HIV-gp120.

### Example 5: Detection of activity of CAR-T cells in inhibiting viral replication in vitro under co-culture conditions

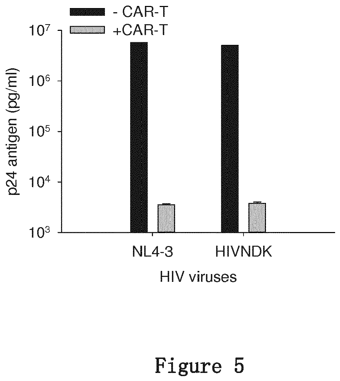

In order to further demonstrate the effectiveness of the N6-CAR-T cells in the clearance of wild-type HIV-1-infected primary CD4+ T cells, the two wild type strains, HIV-1 NL4-3-EGFP and NDK-EGEP, were used to infect CD4+ T lymphocytes isolated from healthy human blood samples, and the cells were changed 3 hours after infection. On the 8th day after infection, the cells were mixed with N6-CAR-modified homologous CD8⁺ T lymphocytes at a ratio of 1:4. The cell killing experiments were performed in a 24-well plate. The target cell number was 10⁶/well and the RMPI 1640 complete medium volume was 500 µl/well. After 48 hours, the ratio of EGFP+CD4+ T lymphocytes was detected by flow cytometry, and the killing effect of the N6-CAR-T cells was verified. The experimental results are shown in FIG. 5.

The results showed that, with the CD8⁺ T cell group modified without CAR molecule as a reference, the N6-CAR-T cell group could clear 99.5% of HIV-1-infected cells and showed a significant killing effect, which fully demonstrated the specificity and efficiency of the N6-CAR-T cells.

### Example 6: Detection of activity of CAR-T lymphocytes in killing HIV virus-infected cells in vivo

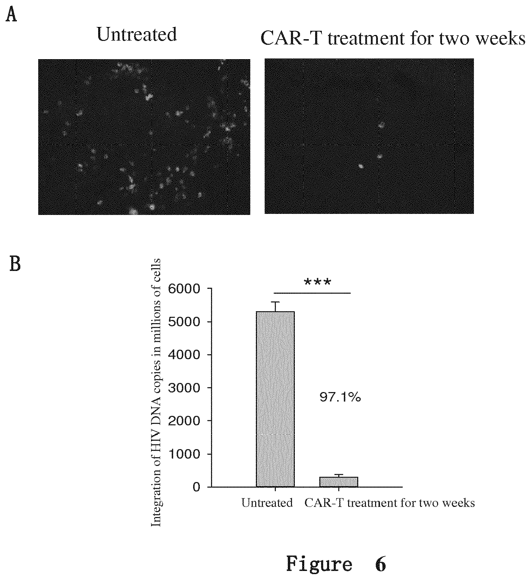

In order to further demonstrate whether the Anti-gp120 CAR-T lymphocytes could clear HIV-infected cells in vivo, the NL4-3-EGFP virus (1x10⁶ pg p24/mouse) carrying the fluorescent gene was intravenously injected into humanized mice BLT, and while infecting the mice, PBMC was isolated from healthy volunteers and then CD8⁺ T cells were isolated, which was transduced with N6-CAR lentivirus, expanded in vitro for 10 days, counted and then resuspended in 500 µl of PBS, and intravenously reinfused at a dose of 1 x 10⁷ CD8 + T/kg. Two weeks later, the spleens were collected from the mice and placed in an embedding medium to prepare frozen sections. More than 20 frozen sections with a thickness of 10 µm were prepared, and photographed under a fluorescence confocal microscope. The photographs were quantitatively analyzed using Velocity 5.0 software (FIG. 6A). At the same time, a single cell suspension was prepared using the collected spleen cells, and 5x10⁶ cells were taken to extract genomic DNA using DNAzol. The number of copies of the provirus was quantified using Nested-QPCR to estimate the number of all HIV-infected cells in the body (FIG. 6B).

The results showed that, compared with the control group that did not receive CAR-T cell therapy, the viral protein expression level and the viral genome level were significantly reduced by 97.1%, which confirmed that the CAR-T cells can effectively lyse and clear HIV-infected cells in vivo, laying a theoretical foundation for human clinical testing of the CAR-T cells.

## Claims

1. A single-chain antibody ScFv, **characterized in that**, the single-chain antibody is capable of recognizing gp120 on the surface of an HIV virus-infected cell, is obtained by tandemly ligating light chain and heavy chain variable regions of an antibody against gp120 on the surface of the HIV virus-infected cell, and serves as an extracellular binding domain of an entire CAR molecule, and the amino acid sequence thereof is shown in SEQ ID NO. 2.

2. A gene encoding the single-chain antibody ScFv of claim 1, **characterized in that**, the nucleotide sequence thereof is shown in SEQ ID NO. 1.

3. A chimeric antigen receptor N6-CAR for treating HIV infection, **characterized in that**, the chimeric antigen receptor is obtained by sequentially splicing a signal peptide, the single-chain antibody ScFv of claim 1, CD8 hinge, transmembrane domain of cluster of differentiation CD28-TM and intracellular domain (ICD) thereof, 4-1BB and ζ chain of cluster of differentiation 3 CD3 from N-terminal to C-terminal, and the amino acid sequence of the obtained chimeric antigen receptor is as shown in SEQ ID NO. 4.

4. The gene encoding the chimeric antigen receptor of claim 3, **characterized in that**, the nucleotide sequence thereof is shown in SEQ ID NO. 3.

5. An expression vector containing and capable of expressing an encoding gene of a chimeric antigen receptor N6-CAR having the amino acid sequence of SEQ ID NO. 4, **characterized in that**, the vector is a PTK-EF1α-N6 vector obtained by transformation by using a PTK881 vector as a backbone and replacing a CMV promoter with an EF-1α promoter, and the nucleotide sequence thereof is shown in SEQ ID NO. 5.

6. A genetically modified CD8⁺ T lymphocyte, **characterized by** being a genetically engineered T-lymphocyte capable of expressing a chimeric antigen receptor obtained by transducing a lentiviral vector of a 293T cell transfected with a PTK-EF1α-N6 expression vector into a CD8⁺ T lymphocyte.

7. The genetically modified CD8⁺ T cell of claim 6, **characterized by** being prepared by the following methods: (1) isolating PBMC from peripheral blood and then using magnetic beads for positive screen to obtain CD8⁺ T cells, stimulating with anti-CD3/28 magnetic beads (ratio of cell to magnetic bead: 1:3) for 12 hours, adding a lentivirus recombinant with N6-CAR molecule for infection for 4 hours, and then supplementing fluid (MOI = 5); starting from the third day after virus infection, counting cells and supplementing the culture medium according to cell state and proliferation, adjusting the cell concentration to 0.6 x 10⁶/ml, and supplementing 100 U/mL IL-2, and further expanding the cells until the number of cells for reinfusion is met.

8. Use of N6-CAR gene-modified CD8⁺ T lymphocyte, **characterized in that**, the genetically modified CD8⁺ T lymphocyte is used to prepare a live cell drug against HIV infection.


SEQUENCE LISTING
<110>  Wuhan Bio-raid Biotechnology Co.,Ltd
<120>  Construction and Application of Recombinant Gene for Chimeric Antigen
Receptor for Treating HIV Infection
<130>  2017
<160>  6
<170>  PatentIn version 3.3
<210>  1
<211>  786
<212>  DNA
<213>  N6
<400>  1
atgctgctgc  tggtgaccag cctgctgctg tgcgagctgc cccaccccgc ccccctgctg    60
atcccccgag cgcacctggt acaatcaggg actgcgatga agaaaccggg ggcctcagta    120
agagtctcct  gccagacctc tggatacacc tttaccgccc acatattatt  ttggttccga    180
caggcccccg ggcgaggact tgagtgggtg gggtggatca agccacaata tggggccgtg   240
aattttggtg  gtggttttcg  ggacagggtc acattgactc gagacgtata tagagagatt    300
gcgtacatgg acatcagagg ccttaaacct gacgacacgg ccgtctatta ctgtgcgaga    360
gaccgttcct atggcgactc ctcttgggcc ttagatgcct ggggacaggg aacgacggtc    420
gtcgtctccg cgggcggagg gggttcaggt ggaggaggct ctggcggtgg cggaagctac   480
atccacgtga cccagtctcc gtcctccctg tctgtgtcta ttggagacag agtcaccatc    540
aattgccaga cgagtcaggg tgttggcagt gacctacatt ggtatcaaca caaaccgggg    600
agagccccta aactcttgat ccaccatacc tcttctgtgg aagacggtgt cccctcaaga    660
ttcagcggct ctggatttca cacatctttt aatctgacca tcagcgacct acaggctgac    720
gacattgcca catattactg tcaagtttta caatttttcg gccgagggag tcgactccat    780
attaaa                                                              786

<210>  2
<211>  262
<212>  PRT
<213>  Artificial sequence
<400>  2
Met Leu Leu Leu Val Thr Ser Leu Leu Leu Cys Glu Leu Pro His Pro
1               5                   10                  15
Ala Phe Leu Leu Ile Pro Arg Ala His Leu Val Gln Ser Gly Thr Ala
            20                  25                  30
Met Lys Lys Pro Gly Ala Ser Val Arg Val Ser Cys Gln Thr Ser Gly
        35                  40                  45
Tyr Thr Phe Thr Ala His Ile Leu Phe Trp Phe Arg Gln Ala Pro Gly
    50                  55                  60
Arg Gly Leu Glu Trp Val Gly Trp Ile Lys Pro Gln Tyr Gly Ala Val
65                  70                  75                  80
Asn Phe Gly Gly Gly Phe Arg Asp Arg Val Thr Leu Thr Arg Asp Val
                85                  90                  95
Tyr Arg Glu Ile Ala Tyr Met Asp Ile Arg Gly Leu Lys Pro Asp Asp
            100                 105                 110

Thr Ala Val Tyr Tyr Cys Ala Arg Asp Arg Ser Tyr Gly Asp Ser Ser
        115                 120                 125
Trp Ala Leu Asp Ala Trp Gly Gln Gly Thr Thr Val Val Val Ser Ala
    130                 135                 140
Gly Gly Gly Gly Ser Gly Gly Gly Gly Ser Gly Gly Gly Gly Ser Tyr
145                 150                 155                 160
Ile His Val Thr Gln Ser Pro Ser Ser Leu Ser Val Ser Ile Gly Asp
                165                 170                 175
Arg Val Thr Ile Asn Cys Gln Thr Ser Gln Gly Val Gly Ser Asp Leu
            180                 185                 190
His Trp Tyr Gln His Lys Pro Gly Arg Ala Pro Lys Leu Leu Ile His
        195                 200                 205
His Thr Ser Ser Val Glu Asp Gly Val Pro Ser Arg Phe Ser Gly Ser
    210                 215                 220
Gly Phe His Thr Ser Phe Asn Leu Thr Ile Ser Asp Leu Gln Ala Asp
225                 230                 235                 240

Asp Ile Ala Thr Tyr Tyr Cys Gln Val Leu Gln Phe Phe Gly Arg Gly
                245                 250                 255
Ser Arg Leu His Ile Lys
            260

<210>  3
<211>  1590
<212>  DNA
<213>  Artificial sequence
<400>  3
atgctgctgc tggtgaccag cctgctgctg tgcgagctgc cccaccccgc ccccctgctg      60
atcccccgag cgcacctggt acaatcaggg actgcgatga agaaaccggg ggcctcagta     120
agagtctcct gccagacctc tggatacacc tttaccgccc acatattatt ttggttccga     180
caggcccccg ggcgaggact tgagtgggtg gggtggatca agccacaata tggggccgtg     240
aattttggtg gtggttttcg ggacagggtc acattgactc gagacgtata tagagagatt     300
gcgtacatgg acatcagagg ccttaaacct gacgacacgg ccgtctatta ctgtgcgaga     360
gaccgttcct atggcgactc ctcttgggcc ttagatgcct ggggacaggg aacgacggtc     420
gtcgtctccg cgggcggagg gggttcaggt ggaggaggct ctggcggtgg cggaagctac     480
atccacgtga cccagtctcc gtcctccctg tctgtgtcta ttggagacag agtcaccatc     540
aattgccaga cgagtcaggg tgttggcagt gacctacatt ggtatcaaca caaaccgggg     600
agagccccta aactcttgat ccaccatacc tcttctgtgg aagacggtgt cccctcaaga     660
ttcagcggct ctggatttca cacatctttt aatctgacca tcagcgacct acaggctgac     720
gacattgcca catattactg tcaagtttta caatttttcg gccgagggag tcgactccat     780
attaaaacca cgacgccagc gccgcgacca ccaacaccgg cgcccaccat cgcgtcgcag     840
cccctgtccc tgcgcccaga ggcgtgccgg ccagcggcgg ggggcgcagt gcacacgagg     900
gggctggact tcgcctgtga tttttgggtg ctggtggtgg ttggtggagt cctggcttgc     960
tatagcttgc tagtaacagt ggcctttatt attttctggg tgaggagtaa gaggagcagg    1020
ctcctgcaca gtgactacat gaacatgact ccccgccgcc ccgggcccac ccgcaagcat    1080
taccagccct atgccccacc acgcgacttc gcagcctatc gctccaaacg gggcagaaag    1140
aaactcctgt atatattcaa acaaccattt atgagaccag tacaaactac tcaagaggaa    1200
gatggctgta gctgccgatt tccagaagaa gaagaaggag gatgtgaact gagagtgaag    1260
ttcagcagga gcgcagacgc ccccgcgtac cagcagggcc agaaccagct ctataacgag    1320
ctcaatctag gacgaagaga ggagtacgat gttttggaca agagacgtgg ccgggaccct    1380
gagatggggg gaaagccgag aaggaagaac cctcaggaag gcctgtacaa tgaactgcag    1440
aaagataaga tggcggaggc ctacagtgag attgggatga aaggcgagcg ccggaggggc    1500
aaggggcacg atggccttta ccagggtctc agtacagcca ccaaggacac ctacgacgcc    1560
cttcacatgc aggccctgcc ccctcgctaa                                     1590

<210>  4
<211>  529
<212>  PRT
<213>  Artificial sequence
<400>  4
Met Leu Leu Leu Val Thr Ser Leu Leu Leu Cys Glu Leu Pro His Pro
1               5                   10                  15
Ala Phe Leu Leu Ile Pro Arg Ala His Leu Val Gln Ser Gly Thr Ala
            20                  25                  30
Met Lys Lys Pro Gly Ala Ser Val Arg Val Ser Cys Gln Thr Ser Gly
        35                  40                  45
Tyr Thr Phe Thr Ala His Ile Leu Phe Trp Phe Arg Gln Ala Pro Gly
    50                  55                  60
Arg Gly Leu Glu Trp Val Gly Trp Ile Lys Pro Gln Tyr Gly Ala Val
65                  70                  75                  80
Asn Phe Gly Gly Gly Phe Arg Asp Arg Val Thr Leu Thr Arg Asp Val
                85                  90                  95
Tyr Arg Glu Ile Ala Tyr Met Asp Ile Arg Gly Leu Lys Pro Asp Asp
            100                 105                 110
Thr Ala Val Tyr Tyr Cys Ala Arg Asp Arg Ser Tyr Gly Asp Ser Ser
    115                 120                 125
Trp Ala Leu Asp Ala Trp Gly Gln Gly Thr Thr Val Val Val Ser Ala
    130                 135                 140
Gly Gly Gly Gly Ser Gly Gly Gly Gly Ser Gly Gly Gly Gly Ser Tyr
145                 150                 155                 160

Ile His Val Thr Gln Ser Pro Ser Ser Leu Ser Val Ser Ile Gly Asp
            165                 170                 175
Arg Val Thr Ile Asn Cys Gln Thr Ser Gln Gly Val Gly Ser Asp Leu
        180                 185                 190
His Trp Tyr Gln His Lys Pro Gly Arg Ala Pro Lys Leu Leu Ile His
    195                 200                 205
His Thr Ser Ser Val Glu Asp Gly Val Pro Ser Arg Phe Ser Gly Ser
  210                 215                 220
Gly Phe His Thr Ser Phe Asn Leu Thr Ile Ser Asp Leu Gln Ala Asp
225                 230                 235                 240
Asp Ile Ala Thr Tyr Tyr Cys Gln Val Leu Gln Phe Phe Gly Arg Gly
                245                 250                 255

Ser Arg Leu His Ile Lys Thr Thr Thr Pro Ala Pro Arg Pro Pro Thr
            260                 265                 270
Pro Ala Pro Thr Ile Ala Ser Gln Pro Leu Ser Leu Arg Pro Glu Ala
        275                 280                 285
Cys Arg Pro Ala Ala Gly Gly Ala Val His Thr Arg Gly Leu Asp Phe
    290                 295                 300
Ala Cys Asp Phe Trp Val Leu Val Val Val Gly Gly Val Leu Ala Cys
305                 310                 315                 320
Tyr Ser Leu Leu Val Thr Val Ala Phe Ile Ile Phe Trp Val Arg Ser
                325                 330                 335
Lys Arg Ser Arg Leu Leu His Ser Asp Tyr Met Asn Met Thr Pro Arg
            340                 345                 350
Arg Pro Gly Pro Thr Arg Lys His Tyr Gln Pro Tyr Ala Pro Pro Arg
        355                 360                 365
Asp Phe Ala Ala Tyr Arg Ser Lys Arg Gly Arg Lys Lys Leu Leu Tyr
    370                 375                 380
Ile Phe Lys Gln Pro Phe Met Arg Pro Val Gln Thr Thr Gln Glu Glu
385                 390                 395                 400
Asp Gly Cys Ser Cys Arg Phe Pro Glu Glu Glu Glu Gly Gly Cys Glu
                405                 410                 415
Leu Arg Val Lys Phe Ser Arg Ser Ala Asp Ala Pro Ala Tyr Gln Gln
            420                 425                 430


Gly Gln Asn Gln Leu Tyr Asn Glu Leu Asn Leu Gly Arg Arg Glu Glu
        435                 440                 445
Tyr Asp Val Leu Asp Lys Arg Arg Gly Arg Asp Pro Glu Met Gly Gly
    450                 455                 460
Lys Pro Arg Arg Lys Asn Pro Gln Glu Gly Leu Tyr Asn Glu Leu Gln
465                 470                 475                 480
Lys Asp Lys Met Ala Glu Ala Tyr Ser Glu Ile Gly Met Lys Gly Glu
                485                 490                 495
Arg Arg Arg Gly Lys Gly His Asp Gly Leu Tyr Gln Gly Leu Ser Thr
            500                 505                 510
Ala Thr Lys Asp Thr Tyr Asp Ala Leu His Met Gln Ala Leu Pro Pro
        515                 520                 525
Arg

<210>  5
<211>  9251
<212>  DNA
<213>  Artificial sequence
<400>  5
gacggatcgg gagatctccc gatcccctat ggtcgactct cagtacaatc tgctctgatg        60
ccgcatagtt aagccagtat ctgctccctg cttgtgtgtt ggaggtcgct gagtagtgcg       120
cgagcaaaat ttaagctaca acaaggcaag gcttgaccga caattgcatg aagaatctgc      180

ttagggttag gcgttttgcg ctgcttcgcg atgtacgggc cagatatacg cgttgacatt        240
gattattgac tagttattaa tagtaatcaa ttacggggtc attagttcat agcccatata
300

tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggac tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagcgcgtt ttgcctgtac tgggtctctc 840
tggttagacc agatctgagc ctgggagctc tctggctaac tagggaaccc actgcttaag 900
cctcaataaa gcttgccttg agtgcttcaa gtagtgtgtg cccgtctgtt gtgtgactct 960
ggtaactaga gatccctcag acccttttag tcagtgtgga aaatctctag cagtggcgcc 1020
cgaacaggga cctgaaagcg aaagggaaac cagagctctc tcgacgcagg actcggcttg 1080
ctgaagcgcg cacggcaaga ggcgaggggc ggcgactggt gagtacgcca aaaattttga 1140
ctagcggagg ctagaaggag agagatgggt gcgagagcgt cagtattaag cgggggagaa 1200
ttagatcgcg atgggaaaaa attcggttaa ggccaggggg aaagaaaaaa tataaattaa 1260
aacatatagt atgggcaagc agggagctag aacgattcgc agttaatcct ggcctgttag 1320
aaacatcaga aggctgtaga caaatactgg gacagctaca accatccctt cagacaggat 1380
cagaagaact tagatcatta tataatacag tagcaaccct ctattgtgtg catcaaagga 1440
tagagataaa agacaccaag gaagctttag acaagataga ggaagagcaa aacaaaagta 1500
agaccaccgc acagcaagcg gccgctgatc ttcagacctg gaggaggaga tatgagggac 1560
aattggagaa gtgaattata taaatataaa gtagtaaaaa ttgaaccatt aggagtagca 1620
cccaccaagg caaagagaag agtggtgcag agagaaaaaa gagcagtggg aataggagct 1680
ttgttccttg ggttcttggg agcagcagga agcactatgg gcgcagcctc aatgacgctg 1740
acggtacagg ccagacaatt attgtctggt atagtgcagc agcagaacaa tttgctgagg 1800
gctattgagg cgcaacagca tctgttgcaa ctcacagtct ggggcatcaa gcagctccag 1860
gcaagaatcc tggctgtgga aagataccta aaggatcaac agctcctggg gatttggggt 1920
tgctctggaa aactcatttg caccactgct gtgccttgga atgctagttg gagtaataaa 1980
tctctggaac agatctggaa tcacacgacc tggatggagt gggacagaga aattaacaat 2040
tacacaagct taatacactc cttaattgaa gaatcgcaaa accagcaaga aaagaatgaa 2100
caagaattat tggaattaga taaatgggca agtttgtgga attggtttaa cataacaaat 2160
tggctgtggt atataaaatt attcataatg atagtaggag gcttggtagg tttaagaata 2220
gtttttgctg tactttctat agtgaataga gttaggcagg gatattcacc attatcgttt
2280
cagacccacc tcccaacccc gaggggaccc gacaggcccg aaggaataga agaagaaggt 2340
ggagagagag acagagacag atccattcga ttagtgaacg gatcttccat cgaattcctg 2400
cagcccgggg gatctaaaag aaaagggggg attggggggt acagtgcagg ggaaagaata 2460
gtagacataa tagcaacaga catacaaact aaagaattac aaaaacaaat tacaaaaatt 2520
caaaattttc gggtttatta cagggacagc agagatccag tttatcgatg agtaattcat 2580
acaaaaggac tcgcccctgc cttggggaat cccagggacc gtcgttaaac tcccactaac 2640
gtagaaccca gagatcgctg cgttcccgcc ccctcacccg cccgctctcg tcatcactga 2700
ggtggagaag agcatgcgtg aggctccggt gcccgtcagt gggcagagcg cacatcgccc 2760
acagtccccg agaagttggg gggaggggtc ggcaattgaa ccggtgccta gagaaggtgg 2820
cgcggggtaa actgggaaag tgatgtcgtg tactggctcc gcctttttcc cgagggtggg 2880
ggagaaccgt atataagtgc agtagtcgcc gtgaacgttc tttttcgcaa cgggtttgcc 2940
gccagaacac aggtaagtgc cgtgtgtggt tcccgcgggc ctggcctctt tacgggttat 3000
ggcccttgcg tgccttgaat tacttccacg cccctggctg cagtacgtga ttcttgatcc 3060
cgagcttcgg gttggaagtg ggtgggagag ttcgaggcct tgcgcttaag gagccccttc 3120
gcctcgtgct tgagttgagg cctggcttgg gcgctggggc cgccgcgtgc gaatctggtg 3180
gcaccttcgc gcctgtctcg ctgctttcga taagtctcta gccatttaaa atttttgatg
3240
acctgctgcg acgctttttt tctggcaaga tagtcttgta aatgcgggcc aagatctgca 3300
cactggtatt tcggtttttg gggccgcggg cggcgacggg gcccgtgcgt cccagcgcac 3360
atgttcggcg aggcggggcc tgcgagcgcg gccaccgaga atcggacggg ggtagtctca 3420
agctggccgg cctgctctgg tgcctggcct cgcgccgccg tgtatcgccc cgccctgggc 3480
ggcaaggctg gcccggtcgg caccagttgc gtgagcggaa agatggccgc ttcccggccc 3540
tgctgcaggg agctcaaaat ggaggacgcg gcgctcggga gagcgggcgg gtgagtcacc 3600
cacacaaagg aaaagggcct ttccgtcctc agccgtcgct tcatgtgact ccacggagta 3660
ccgggcgccg tccaggcacc tcgattagtt ctcgagcttt tggagtacgt cgtctttagg 3720
ttggggggag gggttttatg cgatggagtt tccccacact gagtgggtgg agactgaagt 3780
taggccagct tggcacttga tgtaattctc cttggaattt gccctttttg agtttggatc 3840
ttggttcatt ctcaagcctc agacagtggt tcaaagtttt tttcttccat ttcaggtgtc 3900
gtgaggatct atttccggtg aattcgccac cacgcgtctg gaacaatcaa cctctggatt 3960

acaaaatttg tgaaagattg actggtattc ttaactatgt tgctcctttt acgctatgtg 4020
gatacgctgc tttaatgcct ttgtatcatg ctattgcttc ccgtatggct ttcattttct 4080
cctccttgta taaatcctgg ttgctgtctc tttatgagga gttgtggccc gttgtcaggc 4140
aacgtggcgt ggtgtgcact gtgtttgctg acgcaacccc cactggttgg ggcattgcca 4200
ccacctgtca gctcctttcc gggactttcg ctttccccct ccctattgcc acggcggaac 4260
tcatcgccgc ctgccttgcc cgctgctgga caggggctcg gctgttgggc actgacaatt 4320
ccgtggtgtt gtcggggaag ctgacgtcct ttccatggct gctcgcctgt gttgccacct 4380
ggattctgcg cgggacgtcc ttctgctacg tcccttcggc cctcaatcca gcggaccttc 4440
cttcccgcgg cctgctgccg gctctgcggc ctcttccgcg tcttcgcctt cgccctcaga 4500
cgagtcggat ctccctttgg gccgcctccc cgcatcgata ccgtcgagac ctggaaaaac 4560
atggagcaat cacaagtagc aacacagcag ctaccaatgc tgcttgtgcc tggctagaag 4620
cacaagagga ggaggaggtg ggttttccag tcacacctca ggtaccttta agaccaatga 4680
cttacaaggc agctgtagat cttagccact ttttaaaaga aaagggggga ctggaagggc 4740
taattcactc ccaacgaaga caagatatcc ttgatctgtg gatctaccac acacaaggct 4800
acttccctga ttggcagaac tacacaccag ggccagggat cagatatcca ctgacctttg 4860
gatggtgcta caagctagta ccagttgagc aagagaaggt agaagaagcc aatgaaggag 4920
agaacacccg cttgttacac cctgtgagcc tgcatgggat ggatgacccg gagagagaag 4980
tattagagtg gaggtttgac agccgcctag catttcatca catggcccga gagctgcatc 5040
cggactgtac tgggtctctc tggttagacc agatctgagc ctgggagctc tctggctaac 5100
tagggaaccc actgcttaag cctcaataaa gcttgccttg agtgcttcaa gtagtgtgtg 5160
cccgtctgtt gtgtgactct ggtaactaga gatccctcag acccttttag tcagtgtgga 5220
aaatctctag cagggcccgt ttaaacccgc tgatcagcct cgactgtgcc ttctagttgc 5280
cagccatctg ttgtttgccc ctcccccgtg ccttccttga ccctggaagg tgccactccc 5340
actgtccttt cctaataaaa tgaggaaatt gcatcgcatt gtctgagtag gtgtcattct 5400
attctggggg gtggggtggg gcaggacagc aagggggagg attgggaaga caatagcagg 5460
catgctgggg atgcggtggg ctctatggct tctgaggcgg aaagaaccag ctggggctct 5520
agggggtatc cccacgcgcc ctgtagcggc gcattaagcg cggcgggtgt ggtggttacg 5580
cgcagcgtga ccgctacact tgccagcgcc ctagcgcccg ctcctttcgc tttcttccct 5640
tcctttctcg ccacgttcgc cggctttccc cgtcaagctc taaatcgggg catcccttta 5700
gggttccgat ttagtgcttt acggcacctc gaccccaaaa aacttgatta gggtgatggt 5760
tcacgtagtg ggccatcgcc ctgatagacg gtttttcgcc ctttgacgtt ggagtccacg 5820
ttctttaata gtggactctt gttccaaact ggaacaacac tcaaccctat ctcggtctat 5880
tcttttgatt tataagggat tttggggatt tcggcctatt ggttaaaaaa tgagctgatt 5940
taacaaaaat ttaacgcgaa ttaattctgt ggaatgtgtg tcagttaggg tgtggaaagt 6000
ccccaggctc cccaggcagg cagaagtatg caaagcatgc atctcaatta gtcagcaacc 6060
aggtgtggaa agtccccagg ctccccagca ggcagaagta tgcaaagcat gcatctcaat 6120
tagtcagcaa ccatagtccc gcccctaact ccgcccatcc cgcccctaac tccgcccagt 6180
tccgcccatt ctccgcccca tggctgacta atttttttta tttatgcaga ggccgaggcc 6240
gcctctgcct ctgagctatt ccagaagtag tgaggaggct tttttggagg cctaggcttt 6300
tgcaaaaagc tcccgggagc ttgtatatcc attttcggat ctgatcagca cgtgttgaca 6360
attaatcatc ggcatagtat atcggcatag tataatacga caaggtgagg aactaaacca 6420
tggccaagtt gaccagtgcc gttccggtgc tcaccgcgcg cgacgtcgcc ggagcggtcg 6480
agttctggac cgaccggctc gggttctccc gggacttcgt ggaggacgac ttcgccggtg 6540
tggtccggga cgacgtgacc ctgttcatca gcgcggtcca ggaccaggtg gtgccggaca 6600
acaccctggc ctgggtgtgg gtgcgcggcc tggacgagct gtacgccgag tggtcggagg 6660
tcgtgtccac gaacttccgg gacgcctccg ggccggccat gaccgagatc ggcgagcagc 6720
cgtgggggcg ggagttcgcc ctgcgcgacc cggccggcaa ctgcgtgcac ttcgtggccg 6780
aggagcagga ctgacacgtg ctacgagatt tcgattccac cgccgccttc tatgaaaggt 6840
tgggcttcgg aatcgttttc cgggacgccg gctggatgat cctccagcgc ggggatctca 6900
tgctggagtt cttcgcccac cccaacttgt ttattgcagc ttataatggt tacaaataaa 6960
gcaatagcat cacaaatttc acaaataaag catttttttc actgcattct agttgtggtt 7020
tgtccaaact catcaatgta tcttatcatg tctgtatacc gtcgacctct agctagagct 7080
tggcgtaatc atggtcatag ctgtttcctg tgtgaaattg ttatccgctc acaattccac 7140
acaacatacg agccggaagc ataaagtgta aagcctgggg tgcctaatga gtgagctaac 7200
tcacattaat tgcgttgcgc tcactgcccg ctttccagtc gggaaacctg tcgtgccagc 7260
tgcattaatg aatcggccaa cgcgcgggga gaggcggttt gcgtattggg cgctcttccg 7320
cttcctcgct cactgactcg ctgcgctcgg tcgttcggct gcggcgagcg gtatcagctc 7380
actcaaaggc ggtaatacgg ttatccacag aatcagggga taacgcagga aagaacatgt 7440
gagcaaaagg ccagcaaaag gccaggaacc gtaaaaaggc cgcgttgctg gcgtttttcc 7500
ataggctccg cccccctgac gagcatcaca aaaatcgacg ctcaagtcag aggtggcgaa 7560
acccgacagg actataaaga taccaggcgt ttccccctgg aagctccctc gtgcgctctc 7620
ctgttccgac cctgccgctt accggatacc tgtccgcctt tctcccttcg ggaagcgtgg 7680
cgctttctca atgctcacgc tgtaggtatc tcagttcggt gtaggtcgtt cgctccaagc 7740

tgggctgtgt gcacgaaccc cccgttcagc ccgaccgctg cgccttatcc ggtaactatc 7800
gtcttgagtc caacccggta agacacgact tatcgccact ggcagcagcc actggtaaca 7860
ggattagcag agcgaggtat gtaggcggtg ctacagagtt cttgaagtgg tggcctaact 7920
acggctacac tagaaggaca gtatttggta tctgcgctct gctgaagcca gttaccttcg 7980
gaaaaagagt tggtagctct tgatccggca aacaaaccac cgctggtagc ggtggttttt 8040
ttgtttgcaa gcagcagatt acgcgcagaa aaaaaggatc tcaagaagat cctttgatct 8100
tttctacggg gtctgacgct cagtggaacg aaaactcacg ttaagggatt ttggtcatga 8160
gattatcaaa aaggatcttc acctagatcc ttttaaatta aaaatgaagt tttaaatcaa 8220
tctaaagtat atatgagtaa acttggtctg acagttacca atgcttaatc agtgaggcac 8280
ctatctcagc gatctgtcta tttcgttcat ccatagttgc ctgactcccc gtcgtgtaga 8340
taactacgat acgggagggc ttaccatctg gccccagtgc tgcaatgata ccgcgagacc 8400
cacgctcacc ggctccagat ttatcagcaa taaaccagcc agccggaagg gccgagcgca 8460
gaagtggtcc tgcaacttta tccgcctcca tccagtctat taattgttgc cgggaagcta 8520
gagtaagtag ttcgccagtt aatagtttgc gcaacgttgt tgccattgct acaggcatcg 8580
tggtgtcacg ctcgtcgttt ggtatggctt cattcagctc cggttcccaa cgatcaaggc 8640
gagttacatg atcccccatg ttgtgcaaaa aagcggttag ctccttcggt cctccgatcg 8700
ttgtcagaag taagttggcc gcagtgttat cactcatggt tatggcagca ctgcataatt 8760
ctcttactgt catgccatcc gtaagatgct tttctgtgac tggtgagtac tcaaccaagt 8820
cattctgaga atagtgtatg cggcgaccga gttgctcttg cccggcgtca atacgggata 8880
ataccgcgcc acatagcaga actttaaaag tgctcatcat tggaaaacgt tcttcggggc 8940
gaaaactctc aaggatctta ccgctgttga gatccagttc gatgtaaccc actcgtgcac 9000
ccaactgatc ttcagcatct tttactttca ccagcgtttc tgggtgagca aaaacaggaa 9060
ggcaaaatgc cgcaaaaaag ggaataaggg cgacacggaa atgttgaata ctcatactct 9120
tcctttttca atattattga agcatttatc agggttattg tctcatgagc ggatacatat 9180
ttgaatgtat ttagaaaaat aaacaaatag gggttccgcg cacatttccc cgaaaagtgc 9240
Cacctgacgt c 9251

<210> 6
<211> 10841
<212> DNA
<213> Artificial sequence
<400> 6
gacggatcgg gagatctccc gatcccctat ggtcgactct cagtacaatc tgctctgatg 60
ccgcatagtt aagccagtat ctgctccctg cttgtgtgtt ggaggtcgct gagtagtgcg 120
cgagcaaaat ttaagctaca acaaggcaag gcttgaccga caattgcatg aagaatctgc 180
ttagggttag gcgttttgcg ctgcttcgcg atgtacgggc cagatatacg cgttgacatt 240
gattattgac tagttattaa tagtaatcaa ttacggggtc attagttcat agcccatata 300
tggagttccg cgttacataa cttacggtaa atggcccgcc tggctgaccg cccaacgacc 360
cccgcccatt gacgtcaata atgacgtatg ttcccatagt aacgccaata gggactttcc 420
attgacgtca atgggtggac tatttacggt aaactgccca cttggcagta catcaagtgt 480
atcatatgcc aagtacgccc cctattgacg tcaatgacgg taaatggccc gcctggcatt 540
atgcccagta catgacctta tgggactttc ctacttggca gtacatctac gtattagtca 600
tcgctattac catggtgatg cggttttggc agtacatcaa tgggcgtgga tagcggtttg 660
actcacgggg atttccaagt ctccacccca ttgacgtcaa tgggagtttg ttttggcacc 720
aaaatcaacg ggactttcca aaatgtcgta acaactccgc cccattgacg caaatgggcg 780
gtaggcgtgt acggtgggag gtctatataa gcagcgcgtt ttgcctgtac tgggtctctc 840
tggttagacc agatctgagc ctgggagctc tctggctaac tagggaaccc actgcttaag 900
cctcaataaa gcttgccttg agtgcttcaa gtagtgtgtg cccgtctgtt gtgtgactct 960
ggtaactaga gatccctcag acccttttag tcagtgtgga aaatctctag cagtggcgcc 1020
cgaacaggga cctgaaagcg aaagggaaac cagagctctc tcgacgcagg actcggcttg 1080
ctgaagcgcg cacggcaaga ggcgaggggc ggcgactggt gagtacgcca aaaattttga 1140
ctagcggagg ctagaaggag agagatgggt gcgagagcgt cagtattaag cgggggagaa 1200
ttagatcgcg atgggaaaaa attcggttaa ggccaggggg aaagaaaaaa tataaattaa 1260
aacatatagt atgggcaagc agggagctag aacgattcgc agttaatcct ggcctgttag 1320
aaacatcaga aggctgtaga caaatactgg gacagctaca accatccctt cagacaggat 1380
cagaagaact tagatcatta tataatacag tagcaaccct ctattgtgtg catcaaagga 1440
tagagataaa agacaccaag gaagctttag acaagataga ggaagagcaa aacaaaagta 1500
agaccaccgc acagcaagcg gccgctgatc ttcagacctg gaggaggaga tatgagggac 1560
aattggagaa gtgaattata taaatataaa gtagtaaaaa ttgaaccatt aggagtagca 1620
cccaccaagg caaagagaag agtggtgcag agagaaaaaa gagcagtggg aataggagct 1680
ttgttccttg ggttcttggg agcagcagga agcactatgg gcgcagcctc aatgacgctg 1740
acggtacagg ccagacaatt attgtctggt atagtgcagc agcagaacaa tttgctgagg 1800
gctattgagg cgcaacagca tctgttgcaa ctcacagtct ggggcatcaa gcagctccag 1860

gcaagaatcc tggctgtgga aagataccta aaggatcaac agctcctggg gatttggggt 1920
tgctctggaa aactcatttg caccactgct gtgccttgga atgctagttg gagtaataaa 1980
tctctggaac agatctggaa tcacacgacc tggatggagt gggacagaga aattaacaat 2040
tacacaagct taatacactc cttaattgaa gaatcgcaaa accagcaaga aaagaatgaa 2100
caagaattat tggaattaga taaatgggca agtttgtgga attggtttaa cataacaaat 2160
tggctgtggt atataaaatt attcataatg atagtaggag gcttggtagg tttaagaata 2220
gtttttgctg tactttctat agtgaataga gttaggcagg gatattcacc attatcgttt 2280
cagacccacc tcccaacccc gaggggaccc gacaggcccg aaggaataga agaagaaggt 2340
ggagagagag acagagacag atccattcga ttagtgaacg gatcttccat cgaattcctg 2400
cagcccgggg gatctaaaag aaaagggggg attggggggt acagtgcagg ggaaagaata 2460
gtagacataa tagcaacaga catacaaact aaagaattac aaaaacaaat tacaaaaatt 2520
caaaattttc gggtttatta cagggacagc agagatccag tttatcgatg agtaattcat 2580
acaaaaggac tcgcccctgc cttggggaat cccagggacc gtcgttaaac tcccactaac 2640
gtagaaccca gagatcgctg cgttcccgcc ccctcacccg cccgctctcg tcatcactga 2700
ggtggagaag agcatgcgtg aggctccggt gcccgtcagt gggcagagcg cacatcgccc 2760
acagtccccg agaagttggg gggaggggtc ggcaattgaa ccggtgccta gagaaggtgg 2820
cgcggggtaa actgggaaag tgatgtcgtg tactggctcc gcctttttcc cgagggtggg 2880
ggagaaccgt atataagtgc agtagtcgcc gtgaacgttc tttttcgcaa cgggtttgcc 2940
gccagaacac aggtaagtgc cgtgtgtggt tcccgcgggc ctggcctctt tacgggttat 3000
ggcccttgcg tgccttgaat tacttccacg cccctggctg cagtacgtga ttcttgatcc 3060
cgagcttcgg gttggaagtg ggtgggagag ttcgaggcct tgcgcttaag gagccccttc 3120
gcctcgtgct tgagttgagg cctggcttgg gcgctggggc cgccgcgtgc gaatctggtg 3180
gcaccttcgc gcctgtctcg ctgctttcga taagtctcta gccatttaaa atttttgatg 3240
acctgctgcg acgctttttt tctggcaaga tagtcttgta aatgcgggcc aagatctgca 3300
cactggtatt tcggtttttg gggccgcggg cggcgacggg gcccgtgcgt cccagcgcac 3360
atgttcggcg aggcggggcc tgcgagcgcg gccaccgaga atcggacggg ggtagtctca 3420
agctggccgg cctgctctgg tgcctggcct cgcgccgccg tgtatcgccc cgccctgggc 3480
ggcaaggctg gcccggtcgg caccagttgc gtgagcggaa agatggccgc ttcccggccc 3540
tgctgcaggg agctcaaaat ggaggacgcg gcgctcggga gagcgggcgg gtgagtcacc 3600
cacacaaagg aaaagggcct ttccgtcctc agccgtcgct tcatgtgact ccacggagta 3660
ccgggcgccg tccaggcacc tcgattagtt ctcgagcttt tggagtacgt cgtctttagg 3720
ttggggggag gggttttatg cgatggagtt tccccacact gagtgggtgg agactgaagt 3780
taggccagct tggcacttga tgtaattctc cttggaattt gccctttttg agtttggatc 3840
ttggttcatt ctcaagcctc agacagtggt tcaaagtttt tttcttccat ttcaggtgtc 3900
gtgaggatct atttccggtg aattcgccac catgctgctg ctggtgacca gcctgctgct 3960
gtgcgagctg ccccaccccg cccccctgct gatcccccga gcgcacctgg tacaatcagg 4020
gactgcgatg aagaaaccgg gggcctcagt aagagtctcc tgccagacct ctggatacac 4080
ctttaccgcc cacatattat tttggttccg acaggccccc gggcgaggac ttgagtgggt 4140
ggggtggatc aagccacaat atggggccgt gaattttggt ggtggttttc gggacagggt 4200
cacattgact cgagacgtat atagagagat tgcgtacatg gacatcagag gccttaaacc 4260
tgacgacacg gccgtctatt actgtgcgag agaccgttcc tatggcgact cctcttgggc 4320
cttagatgcc tggggacagg gaacgacggt cgtcgtctcc gcgggcggag ggggttcagg 4380
tggaggaggc tctggcggtg gcggaagcta catccacgtg acccagtctc cgtcctccct 4440
gtctgtgtct attggagaca gagtcaccat caattgccag acgagtcagg gtgttggcag 4500
tgacctacat tggtatcaac acaaaccggg gagagcccct aaactcttga tccaccatac 4560
ctcttctgtg gaagacggtg tcccctcaag attcagcggc tctggatttc acacatcttt 4620
taatctgacc atcagcgacc tacaggctga cgacattgcc acatattact gtcaagtttt 4680
acaatttttc ggccgaggga gtcgactcca tattaaaacc acgacgccag cgccgcgacc 4740
accaacaccg gcgcccacca tcgcgtcgca gcccctgtcc ctgcgcccag aggcgtgccg 4800
gccagcggcg gggggcgcag tgcacacgag ggggctggac ttcgcctgtg atttttgggt 4860
gctggtggtg gttggtggag tcctggcttg ctatagcttg ctagtaacag tggcctttat 4920
tattttctgg gtgaggagta agaggagcag gctcctgcac agtgactaca tgaacatgac 4980
tccccgccgc cccgggccca cccgcaagca ttaccagccc tatgccccac cacgcgactt 5040
cgcagcctat cgctccaaac ggggcagaaa gaaactcctg tatatattca aacaaccatt 5100
tatgagacca gtacaaacta ctcaagagga agatggctgt agctgccgat ttccagaaga 5160
agaagaagga ggatgtgaac tgagagtgaa gttcagcagg agcgcagacg cccccgcgta 5220
ccagcagggc cagaaccagc tctataacga gctcaatcta ggacgaagag aggagtacga 5280
tgttttggac aagagacgtg gccgggaccc tgagatgggg ggaaagccga gaaggaagaa 5340
ccctcaggaa ggcctgtaca atgaactgca gaaagataag atggcggagg cctacagtga 5400
gattgggatg aaaggcgagc gccggagggg caaggggcac gatggccttt accagggtct 5460
cagtacagcc accaaggaca cctacgacgc ccttcacatg caggccctgc cccctcgcta 5520
aacgcgtctg gaacaatcaa cctctggatt acaaaatttg tgaaagattg actggtattc 5580
ttaactatgt tgctcctttt acgctatgtg gatacgctgc tttaatgcct ttgtatcatg 5640

ctattgcttc ccgtatggct ttcattttct cctccttgta taaatcctgg ttgctgtctc 5700
tttatgagga gttgtggccc gttgtcaggc aacgtggcgt ggtgtgcact gtgtttgctg 5760
acgcaacccc cactggttgg ggcattgcca ccacctgtca gctcctttcc gggactttcg 5820
ctttccccct ccctattgcc acggcggaac tcatcgccgc ctgccttgcc cgctgctgga 5880
caggggctcg gctgttgggc actgacaatt ccgtggtgtt gtcggggaag ctgacgtcct 5940
ttccatggct gctcgcctgt gttgccacct ggattctgcg cgggacgtcc ttctgctacg 6000
tcccttcggc cctcaatcca gcggaccttc cttcccgcgg cctgctgccg gctctgcggc 6060
ctcttccgcg tcttcgcctt cgccctcaga cgagtcggat ctccctttgg gccgcctccc 6120
cgcatcgata ccgtcgagac ctggaaaaac atggagcaat cacaagtagc aacacagcag 6180
ctaccaatgc tgcttgtgcc tggctagaag cacaagagga ggaggaggtg ggttttccag 6240
tcacacctca ggtaccttta agaccaatga cttacaaggc agctgtagat cttagccact 6300
ttttaaaaga aaagggggga ctggaagggc taattcactc ccaacgaaga caagatatcc 6360
ttgatctgtg gatctaccac acacaaggct acttccctga ttggcagaac tacacaccag 6420
ggccagggat cagatatcca ctgacctttg gatggtgcta caagctagta ccagttgagc 6480
aagagaaggt agaagaagcc aatgaaggag agaacacccg cttgttacac cctgtgagcc 6540
tgcatgggat ggatgacccg gagagagaag tattagagtg gaggtttgac agccgcctag 6600
catttcatca catggcccga gagctgcatc cggactgtac tgggtctctc tggttagacc 6660
agatctgagc ctgggagctc tctggctaac tagggaaccc actgcttaag cctcaataaa 6720
gcttgccttg agtgcttcaa gtagtgtgtg cccgtctgtt gtgtgactct ggtaactaga 6780
gatccctcag acccttttag tcagtgtgga aaatctctag cagggcccgt ttaaacccgc 6840
tgatcagcct cgactgtgcc ttctagttgc cagccatctg ttgtttgccc ctcccccgtg 6900
ccttccttga ccctggaagg tgccactccc actgtccttt cctaataaaa tgaggaaatt 6960
gcatcgcatt gtctgagtag gtgtcattct attctggggg gtggggtggg gcaggacagc 7020
aagggggagg attgggaaga caatagcagg catgctgggg atgcggtggg ctctatggct 7080
tctgaggcgg aaagaaccag ctggggctct agggggtatc cccacgcgcc ctgtagcggc 7140
gcattaagcg cggcgggtgt ggtggttacg cgcagcgtga ccgctacact tgccagcgcc 7200
ctagcgcccg ctcctttcgc tttcttccct tcctttctcg ccacgttcgc cggctttccc 7260
cgtcaagctc taaatcgggg catcccttta gggttccgat ttagtgcttt acggcacctc 7320
gaccccaaaa aacttgatta gggtgatggt tcacgtagtg ggccatcgcc ctgatagacg 7380
gtttttcgcc ctttgacgtt ggagtccacg ttctttaata gtggactctt gttccaaact 7440
ggaacaacac tcaaccctat ctcggtctat tcttttgatt tataagggat tttggggatt 7500
tcggcctatt ggttaaaaaa tgagctgatt taacaaaaat ttaacgcgaa ttaattctgt 7560
ggaatgtgtg tcagttaggg tgtggaaagt ccccaggctc cccaggcagg cagaagtatg 7620
caaagcatgc atctcaatta gtcagcaacc aggtgtggaa agtccccagg ctccccagca 7680
ggcagaagta tgcaaagcat gcatctcaat tagtcagcaa ccatagtccc gcccctaact 7740
ccgcccatcc cgcccctaac tccgcccagt tccgcccatt ctccgcccca tggctgacta 7800
atttttttta tttatgcaga ggccgaggcc gcctctgcct ctgagctatt ccagaagtag 7860
tgaggaggct tttttggagg cctaggcttt tgcaaaaagc tcccgggagc ttgtatatcc 7920
attttcggat ctgatcagca cgtgttgaca attaatcatc ggcatagtat atcggcatag 7980
tataatacga caaggtgagg aactaaacca tggccaagtt gaccagtgcc gttccggtgc 8040
tcaccgcgcg cgacgtcgcc ggagcggtcg agttctggac cgaccggctc gggttctccc 8100
gggacttcgt ggaggacgac ttcgccggtg tggtccggga cgacgtgacc ctgttcatca 8160
gcgcggtcca ggaccaggtg gtgccggaca acaccctggc ctgggtgtgg gtgcgcggcc 8220
tggacgagct gtacgccgag tggtcggagg tcgtgtccac gaacttccgg gacgcctccg 8280
ggccggccat gaccgagatc ggcgagcagc cgtgggggcg ggagttcgcc ctgcgcgacc 8340
cggccggcaa ctgcgtgcac ttcgtggccg aggagcagga ctgacacgtg ctacgagatt 8400
tcgattccac cgccgccttc tatgaaaggt tgggcttcgg aatcgttttc cgggacgccg 8460
gctggatgat cctccagcgc ggggatctca tgctggagtt cttcgcccac cccaacttgt 8520
ttattgcagc ttataatggt tacaaataaa gcaatagcat cacaaatttc acaaataaag 8580
catttttttc actgcattct agttgtggtt tgtccaaact catcaatgta tcttatcatg 8640
tctgtatacc gtcgacctct agctagagct tggcgtaatc atggtcatag ctgtttcctg 8700
tgtgaaattg ttatccgctc acaattccac acaacatacg agccggaagc ataaagtgta 8760
aagcctgggg tgcctaatga gtgagctaac tcacattaat tgcgttgcgc tcactgcccg 8820
ctttccagtc gggaaacctg tcgtgccagc tgcattaatg aatcggccaa cgcgcgggga 8880
gaggcggttt gcgtattggg cgctcttccg cttcctcgct cactgactcg ctgcgctcgg 8940
tcgttcggct gcggcgagcg gtatcagctc actcaaaggc ggtaatacgg ttatccacag 9000
aatcagggga taacgcagga aagaacatgt gagcaaaagg ccagcaaaag gccaggaacc 9060
gtaaaaaggc cgcgttgctg gcgtttttcc ataggctccg cccccctgac gagcatcaca 9120
aaaatcgacg ctcaagtcag aggtggcgaa acccgacagg actataaaga taccaggcgt 9180
ttccccctgg aagctccctc gtgcgctctc ctgttccgac cctgccgctt accggatacc 9240
tgtccgcctt tctcccttcg ggaagcgtgg cgctttctca atgctcacgc tgtaggtatc 9300
tcagttcggt gtaggtcgtt cgctccaagc tgggctgtgt gcacgaaccc cccgttcagc 9360
ccgaccgctg cgccttatcc ggtaactatc gtcttgagtc caacccggta agacacgact 9420

tatcgccact ggcagcagcc actggtaaca ggattagcag agcgaggtat gtaggcggtg 9480
ctacagagtt cttgaagtgg tggcctaact acggctacac tagaaggaca gtatttggta 9540
tctgcgctct gctgaagcca gttaccttcg gaaaaagagt tggtagctct tgatccggca 9600
aacaaaccac cgctggtagc ggtggttttt ttgtttgcaa gcagcagatt acgcgcagaa 9660
aaaaaggatc tcaagaagat cctttgatct tttctacggg gtctgacgct cagtggaacg 9720
aaaactcacg ttaagggatt ttggtcatga gattatcaaa aaggatcttc acctagatcc 9780
ttttaaatta aaaatgaagt tttaaatcaa tctaaagtat atatgagtaa acttggtctg 9840
acagttacca atgcttaatc agtgaggcac ctatctcagc gatctgtcta tttcgttcat 9900
ccatagttgc ctgactcccc gtcgtgtaga taactacgat acgggagggc ttaccatctg 9960
gccccagtgc tgcaatgata ccgcgagacc cacgctcacc ggctccagat ttatcagcaa 10020
taaaccagcc agccggaagg gccgagcgca gaagtggtcc tgcaacttta tccgcctcca 10080
tccagtctat taattgttgc cgggaagcta gagtaagtag ttcgccagtt aatagtttgc 10140
gcaacgttgt tgccattgct acaggcatcg tggtgtcacg ctcgtcgttt ggtatggctt 10200
cattcagctc cggttcccaa cgatcaaggc gagttacatg atcccccatg ttgtgcaaaa 10260
aagcggttag ctccttcggt cctccgatcg ttgtcagaag taagttggcc gcagtgttat 10320
cactcatggt tatggcagca ctgcataatt ctcttactgt catgccatcc gtaagatgct 10380
tttctgtgac tggtgagtac tcaaccaagt cattctgaga atagtgtatg cggcgaccga 10440
gttgctcttg cccggcgtca atacgggata ataccgcgcc acatagcaga actttaaaag 10500
tgctcatcat tggaaaacgt tcttcggggc gaaaactctc aaggatctta ccgctgttga 10560
gatccagttc gatgtaaccc actcgtgcac ccaactgatc ttcagcatct tttactttca 10620
ccagcgtttc tgggtgagca aaaacaggaa ggcaaaatgc cgcaaaaaag ggaataaggg 10680
cgacacggaa atgttgaata ctcatactct tcctttttca atattattga agcatttatc 10740
agggttattg tctcatgagc ggatacatat ttgaatgtat ttagaaaaat aaacaaatag 10800
gggttccgcg cacatttccc cgaaaagtgc cacctgacgt c 10841